Europäisches Patentamt

European Patent Office

Office européen des brevets

(19)

(11) **EP 1 726 311 A2**

(12) # EUROPEAN PATENT APPLICATION

(43) Date of publication:
**29.11.2006 Bulletin 2006/48**

(51) Int Cl.:
*A61K 38/19* (2006.01)   *A61K 35/28* (2006.01)
*A61P 1/16* (2006.01)

(21) Application number: **06011313.1**

(22) Date of filing: **05.12.2002**

(84) Designated Contracting States:
**AT BE BG CH CY CZ DE DK EE ES FI FR GB GR
IE IT LI LU MC NL PT SE SI SK TR**
Designated Extension States:
**AL LT LV MK RO**

(30) Priority: **06.12.2001 IL 14697001**

(62) Document number(s) of the earlier application(s) in
accordance with Art. 76 EPC:
**02793294.6 / 1 453 533**

(71) Applicant: **Yeda Research and development co.
Ltd.,
an Israeli Company
76100 Rehovot (IL)**

(72) Inventors:
• **Kollet, Orit
52232 Ramat Gan (IL)**
• **Lapidot, Tsvee
74046 Ness Ziona (IL)**

(74) Representative: **Grünecker, Kinkeldey,
Stockmair & Schwanhäusser
Anwaltssozietät
Maximilianstrasse 58
80538 München (DE)**

Remarks:
This application was filed on 31 - 05 - 2006 as a
divisional application to the application mentioned
under INID code 62.

(54) **Migration of hematopoietic stem cells and progenitor cells to the liver**

(57)    The invention relates to transplantation of hematopoietic stem cells (HSC) and/or progenitor cells (HPC) into the liver. More specifically the invention relates to the use of chemokines, preferably SDF-1, for enhancing homing of HSC/HPC to the liver.

EP 1 726 311 A2

**Description**

FIELD OF THE INVENTION

The invention relates to transplantation of hematopoietic stem cells (HSC) and/or progenitor cells (HPC) into the liver. More specifically the invention relates to the use of chemokines, preferably SDF-1, for enhancing homing of HSC/HPC to the liver.

BACKGROUND OF THE INVENTION

Stem cells are capable of self-renewal and division, leading to more stem cells and to differentiated cells. Hematopoietic stem cells (HSC) have the property of giving rise to sufficient hematopoietic activity to rescue a lethally irradiated recipient from hematopoietic failure (Morrison et al.1995).

Bone marrow contains mesenchymal and hematopoietic cells. The mesenchymal stem cells give rise to adipocytic, chondrocytic and osteocytic lineage, including the stromal cells of bone marrow (Pittenger et al. 1999). The hematopoietic stem cells have been found to give rise to lymphoid, myeloid and erythrocytic lineages.

In mouse, HSCs represents a rare population of 0.01 % of whole bone marrow and have been isolated using the combination of markers: $Thy^{low}$ $Lin^{neg}$ $Scal^+$ $ckit^{high}$ (KTLS). In humans CD34+ Thy-1+ Lin- hematopoietic stem cells are the human equivalents of the mouse KTLS hematopoietic stem cells (Ikuta et al 1992). Mammalian hematopoietic cells are described in US5,087,570 and human hematopoietic stem cells in US5,061,620.

The mechanisms that guide circulating hematopoietic progenitor cells (HPC or HSC) are clinically significant because the success of stem cell transplantation depends on efficient targeting of grafted cells in a recipient's bone marrow (Mazo and von Adrian 1999). It is due to this homing of transplanted cells that bone marrow transplantations can be performed by simple intravenous infusion, rather than requiring invasive surgery, as in the case with the transplantation of any other organ. Homing of HPC can be defined as the set of molecular interactions that allows circulating HPC to recognize, adhere to, and migrate across bone marrow endothelial cells and results in the accumulation of HPC in the unique hematopoiesis-promoting microenvironment of the bone marrow. Homing of progenitor cells can be conceived as a multi-step phenomenon. HPC arriving to the bone marrow must first interact with the luminal surface of the bone marrow endothelium. This interaction must occur within seconds after the HPC has entered the bone marrow microvasculature and provide sufficient mechanical strength to permit the adherent cell to withstand the shear force exerted by the flowing blood. Adherent HPC must then pass through the endothelial layer to enter the hematopoietic compartment. After extravasation, HPC encounter specialized stromal cells whose juxtaposition supports maintenance of the immature pool by self-renewal process in addition to lineage-specific HPC differentiation, proliferation and maturation, a process that involves stroma-derived cytokines and other growth signals.

The cDNAs of murine SDF-1-alpha and SDF-1-beta encode proteins of 89 and 93 amino acids, respectively (Cytokines Online Pathfinder Encyclopaedia, www.copewithcytokines.de/cope.cgi). The amino acid sequences are identical, differing only by the presence of 4 additional amino acids at the C-terminus of SDF-1-beta. SDF-1-alpha and SDF-1-beta sequences are more than 92 percent identical to those of the human counterparts. Human SDF-1-alpha and SDF-1-beta are encoded by a single gene and arise by alternative splicing. The human SDF-1 gene is located on chromosome 10q11.1. Peptides corresponding to the N-terminal 9 residues of the factor have been shown to possess activities similar to SDF-1 although the peptides were less potent. The human and mouse SDF-1 were found to be cross-reactive.

The SDF-1 gene is expressed ubiquitously (Cytokines Online Pathfinder Encyclopaedia). SDF-1 acts on a variety of lymphoid and myeloid cells in vitro and is a highly potent chemo attractant for mononuclear cells in vivo. In addition, SDF-1 also induces intracellular actin polymerization in lymphocytes. In vitro and in vivo SDF-1 acts as a chemo attractant for human hematopoietic progenitor cells expressing CD34 (CFU-GEMM, BFU-E , CFU-GM , CFU) and CXCR4 giving rise to mixed types of progenitors, and more primitive types. The chemotactic response is inhibited by pertussis toxin. Chemotaxis of CD34 (+) cells in response to SDF is increased by IL-3 in vitro. SDF has been shown also to induce a transient elevation of cytoplasmic calcium in these cells.

SDF-1 is also called pre-B-cell growth-stimulating factor (PBSF) and has been reported to be a powerful chemo attractant (chemokine) for lymphocytes, monocytes, and primary CD34+cells. SDF-1 is a chemotactic factor that induces migration of cells and the direction of cell movement is determined by the concentration gradient of SDF-1 (Kim and Broxmeyer 1998) low in the peripheral blood and high in the bone marrow. Since SDF-1 is produced by bone marrow stroma cells, it was hypothesized that an SDF-1 gradient is formed between the bone marrow microenvironment to the blood system. This gradient attracts HPC, and retains them in the bone marrow microenvironment, unless, this gradient is broken by administered or induced effectors molecules in the blood.

The receptor of SDF-1, CXCR4, is expressed on many cell types, including bone marrow cells, mobilized bone marrow cells cord blood cells, including the sub population of cord blood CD34+ cell, $CD34^+CD38^-$ cells, which are pluripotent hematopoietic precursor cells. Treatment of the human HPCs, CD34+, with anti CXCR4 antibody before transplantation results in reduction of bone marrow engraftment in NOD/SCID mice (Peled et al Science 1999).

Immature human CD34+ cells and primitive CD34+ICD38-/low cells, which do not migrate toward a gradient of SDF-1 in vitro, and do not home and repopulate in vivo the murine bone marrow, can become functional repopulating cells by short-term 16 to 48 hr in vitro stimulation with cytokines such as SLF and IL-6 prior to transplantation (Kollet et al. 2000, Peled et al. 1999 Lapidot 2001). These cytokines increase surface CXCR4 expression, migration toward SDF-1, and in vivo homing and repopulation.

It has been reported that SDF-1 is also a key factor in stimulation of human stem cell adherence to endothelial cell in the bone marrow microvasculature (Peled et al The Journal of Clinical Investigation 1999). Therefore SDF-1 is implicated not only as chemo attractant for stem and progenitor cells, but also as mediator of integrin dependent cell adhesion and transendothelial migration required for engraftment in the bone marrow.

HPCs can be mobilized from the bone marrow to the peripheral blood in response to injected cytokines such granulocyte-macrophage colony-stimulating factor (GM-CSF), granulocyte colony-stimulating factor (G-CSF), and Steel factor (SLF) [Siena et al, 1989, Duhrsen et al 1988, Drize et al 1996]. Mobilization of stem cells from donor's bone marrow into the blood and their retrieval from the blood, for transplantation procedures, is increasingly being used world wide, it is replacing the recovery of these stem cells from the donor's bone marrow using invasive surgery.

Mobilization allows bone marrow repopulation with own HSC recovered and reserved from patients prior irradiation and chemotherapic treatments (autologous transplantation). The recovery of HSC is greater from mobilization than from a cord blood and bone marrow surgery.

The liver is an organ capable of extensive regeneration. Tissue loss or chemical injury induces release of cytokines such as TNF-$\alpha$ which in conjunction with growth factors (reviewed in Blau HM 2001 Cell 105:829, Bryon E, Blood Cells, Molecules, and Diseases 2001, 27:590), Webber EM, Hepatology 1998 28:1226) trigger liver regeneration.

Currently liver transplantation is the only available therapy for end-stage liver failure. However, many of these patients die every year waiting for suitable histocompatible donor organs.

To study liver regeneration, a simple experimental model of partial hepatectomy was developed in the rat. In these models, 2/3 of the liver mass is removed. Interestingly, less than a week after the operation, the remaining lobes enlarge to replace the lost hepatic tissues (Diehl et al. 1996). These studies established that the regenerative process was almost certainly the result of the proliferation of mature hepatocytes. Later on, cellular therapy has been successfully applied in rodent models using primary hepatocytes, the chief functional cells of the liver (Weglartz et al 2000). Liver regeneration after loss of hepatocytes e.g. caused by food toxins is a fundamental mechanism in response to injury. Clinical studies suggest that hepatocyte transplantation may be useful for bridging patients to whole organ transplantation, for providing metabolic support during liver failure, and for replacing whole organ transplantation in certain metabolic liver diseases (Strom et al. 1997). However, the potential of using hepatocytes is challenged by allograft rejection limits, hepatocyte viability after isolation and the poor cryopreservation of these cells for latter use. Liver stem cells or their progeny would be a better alternative for liver regeneration. It is not known, however, whether stem cells and their progeny participate also in the regenerative process of the liver, and whether they are the only source of sustainable regeneration. Liver stem cells have not been identified yet. It is now accepted that under certain conditions where mature hepatocytes cannot regenerate, stem cells and progenitor cells, perhaps oval cells, will proliferate and eventually differentiate into hepatocytes in the adult liver. Several laboratories have found that within the liver reside clonogenic precursors of both hepatocytes and bile duct progeny (Grisham et al. 1997 and Novikoff et al. 1996).

It has been proposed that the precursor of liver stem cells may reside in another tissue. Petersen et al. have suggested that adult bone marrow is a potential source of oval cells and hepatocytes (Petersen et al 1999). These reports did not establish the nature of the progenitor cells or whether they can reconstitute liver function. Bone marrow is composed of mixed population of cells of different origins. There are at least two types of stem cells residing the bone marrow, HSC, as described above, and the mesenchymal stem cells that can differentiate in a variety of cell types like chondrocytes, osteocytes, and adipocites. In addition, it has been suggested that bone marrow contains precursors of endothelium, skeletal muscle, and brain. Those previous studies do not distinguish whether HSC, mesenchymal stem cells, or as-yet-unknown progenitors residing in the bone marrow are responsible for liver engraftment.

Hepatic injury was induced in female rats transplanted with male bone marrow and treated with a drug, which inhibits hepatocyte proliferation (Petersen et al. 1999). Markers for Y chromosome, dipeptidyl peptidase IV enzyme, and L21-6 antigen were used to identify liver cells of bone marrow origin. It was found that a proportion of the regenerated hepatic cells were donor-derived i.e. of bone marrow origin.

A study has been conducted in female patients who have received a bone-marrow transplant from a male donor (Alison et al. 2000). The presence of the Y chromosome in the liver has been monitored using a DNA specific probe. The fact that Y positive cells were found in the liver indicates an extrahepatic origin for these cells. The hepatic nature of the Y positive cells in the liver was confirmed by their immunoexpression of cytokeratin 8. These results indicate that adult HSC can be found in the liver and are capable to differentiate into hepatocytes.

The authors suggest that bone marrow cells can differentiate into oval cells known to be liver resident and capable under specific physio-pathological conditions to differentiate into the two types of epithelial cells present in the liver: ductular cells and hepatocytes. To support this notion they mention that oval cells have some phenotypic traits that are typical of bone marrow stem cells e.g. the CD34+ marker (Wolfe et al. 1985, Noveli et al 1996, Van Eyken et al 1993 and Tanaka et al 1999).

Using an inducible animal of lethal hereditary liver disease, tyrosinemia type 1, it has been demonstrated that highly purified CD45+ enriched HS C from adult bone marrow have hepatic as well as hematopoietic reconstitution activity (Lagasse et al 2000 b).

The possibility of using hematopoietic stem cell for giving rise to hepatocytes has been reviewed by Lagasse et al. (2001).

WO 01/71016 (Lagasse et al.) discloses methods for the generation of non-hematopoietic tissues from hematopoietic stem cells. More specifically it discloses that HSC transplantation can regenerate hepatocytes.

This extraordinary potential of HSC to generate hepatocytes may have considerable advantages over the use of hepatocytes alone for liver regeneration. Bone marrow can be easily obtained and allows the use of living, related, HLA matched donors and even of own HSC cells. However with current protocols in mice is not feasible since the HSC to hepatocyte transition takes weeks to months.

Thus there exists a need to provide a feasible HSC cell-based therapy method allowing liver regeneration enabling treatment of the increased number of patients in need.

**SUMMARY OF THE INVENTION**

The invention provides the use of one or more chemokines such as SDF-1 or an analog fusion protein variant or fragment thereof, and/or an agent in the manufacture of a medicament for enhancing homing of hematopoietic stem cells (HSC) and/or progenitor cells (HPC) to the liver of a subject in need. Specifically, said HSC/HPC may be allogeneic, syngeneic or autologous and/or embryonic, and/or neonatal e.g. from the human umbilical cord blood, and/or from adult origin e.g. from the bone marrow and/or mobilized peripheral blood. More specifically, the HSC/HPC may be enriched for CD34+ cells and preferably for CD34+/CD38-/low cells.

In addition, the HSC/HPC of the invention may be genetically modified cells producing a therapeutic agent.

Furthermore, the HSC/HPC of the invention may could be pre-treated with a growth factor such as, SFL or IL-6, preferably IL-6 and its receptor, more preferably a chimeric protein comprising IL-6 and its receptor or could be pre-treated with supporting cells.

In addition, the medicament of the invention may further comprise a mobilizing agent such as IL-3, SLF, GM-CSF and preferably G-CSF and /or may further comprise cells which are different from said HSC/HPC e.g. hepatic cells.

In one aspect, the invention relates to the use of a chemokine such SDF-1 in the manufacture of a medicament for enhancing migration of HSC/HPC to the liver of a subject in need. More specifically, for a subject suffering from a liver disease and/or for a subject in need of liver targeted gene therapy e.g. in diseases such as Gaucher disease and Glycogen storage disease.

In another aspect, the invention relates to methods for increasing homing of hematopoietic stem cells (HSC) and/or hematopoietic progenitor cells (HPC) to the liver of a subject in need comprising: administering and/or mobilizing said cells and treating the subject in need with a chemokine, preferably SDF-1 or an analog fusion protein variant or fragment thereof.

BRIEF DESCRIPTION OF THE FIGURES

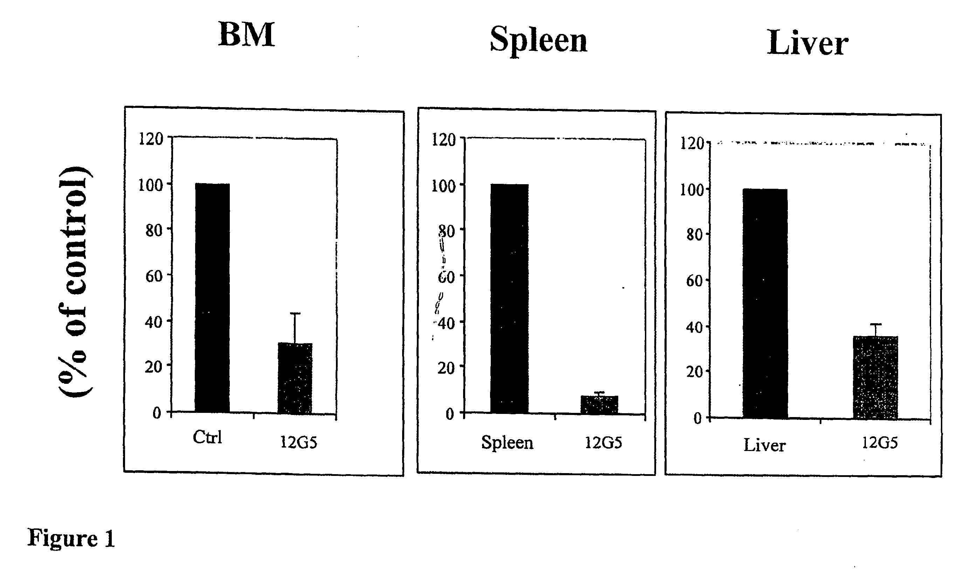

Figure 1 shows that homing of human MPB CD34+ enriched cells into the liver of NOD/SCID mice is CXCR4 dependent.

Human enriched CD34+ mobilized peripheral blood (MPB) cells were incubated and co-transplanted with anti CXCR4 antibody (12G5, 10μg/mouse) or with media alone. 0.5-1x10^6 CD34+ MPB cells were transplanted into NOD/SCID mice by intravenous (IV) injection, 24h. post 375cGy sublethally irradiation. Mice were sacrified 16h later. Single cell suspension were prepared and introduced to flow cytometry (FACS) analysis, using anti human CD34 FITC and anti CD38 PE. n=3 exp. 4 mice/group.

DETAILED DESCRIPTION OF THE INVENTION

The present invention relates to the use of a chemokine, preferably CXC chemokine and more preferably SDF-1 or an analog fusion protein variant or fragment thereof in the manufacture of a medicament for enhancing and/or directing homing of hematopoietic stem cells (HSC) and/or hematopoietic precursor cells (HPC) to the liver of a subject in need.

The present invention is based on the finding that transplanted human HSC and/or progenitor cells migrate directly to the liver and that such migration is mediated by a chemokine such as SDF-1.

More specifically, the invention is based on results obtained using an experimental animal model for migration or homing of HSC. In the experimental model human (donor) HSC e.g. HSC obtained from bone marrow cells, human cord blood cells or mobilized peripheral blood cells, is administered to sub-lethally irradiated non obese diabetes severe combined immune deficient (NOD/SCID) mice (recipient) and after a few hours following cell administration (e.g. 16 hours), the human HSC reaching a specific organ is monitored (Kollet et al 2001). Using such experimental model, it was found for the first time that donor human HSC home directly into the recipient's liver. The results disclosed show also that when the human HSC were pre-treated and co-transplanted with anti-CXCR4 (receptor of SDF-1) neutralizing antibody, homing to the liver was significantly reduced. This results demonstrate that homing of human HSC to the liver requires the activity of CXCR4.

Since irradiation is known to increase bone marrow SDF-1 and consequently to increase HSC migration to bone marrow (Ponomaryov et al 2000), and because the above findings indicate involvement of SDF-1 activity in HSC migration to the liver, the effect of irradiation on HSC migration to the liver was explored. Using the above experimental model, migration of transplanted human HSC to the liver was measured and compared in irradiated versus non-irradiated mice. The results obtained show that migration of human HSC to the liver is significantly reduced in non-irradiated mice. In order to check whether one of the causes of such reduction in migration in non-irradiated mouse is the lack of SDF-1 expression in the liver, migration of human HSC to the liver in non-irradiated mice was measured and compared in mice that were injected with SDF-1 into the liver versus non treated mice. The results obtained show that migration of human HSC to the liver of non-irradiated mice improves significantly with SDF-1 administration. Moreover, co-transplantation of stem cells in non-irradiated mice with anti CXCR4 antibodies inhibited homing in SDF-1 treated mice, demonstrating that homing to the liver is specifically induced by the injected SDF-1 and the interaction with its receptor, CXCR4.

Thus, these finding demonstrate that upon irradiation, SDF-1 is expressed in the liver and that its chemotactic role is needed for the migration of human stem and progenitor cells to the liver. In addition to irradiation, other DNA damaging agents for example cyclophosphamide, and 5-fluorouracil may induce SDF-1 expression in the liver. Also, such DNA damaging agents may induce, in addition to SDF-1, the expression of other chemokines having a role in migration of HSC to the liver.

Within the context of the present invention, the expressions "migration" and "homing" are used synonymously.

Chemo attractants and chemokines are chemotactic factors that induce positive chemotaxis e.g. SDF-1. Chemokines are a family of pro-inflammatory activation-inducible cytokines previously referred to as members of SIS family of cytokines ,SIG family of cytokines ,SCY family of cytokines ,Platelet factor-4 superfamily or Intercrines. These proteins are mainly chemotactic for different cell types (hence the name, which is derived from (chemo)tactic cyto (kines). CXC chemokines e.g. SDF-1 have the first two cysteine residues separated by a single amino acids.

A chemokine, that was tested in vivo assays e.g. the above experimental model, and was found to positively affect migration of HSC to the liver, like SDF-1, could be used according to the invention. For example, a candidate chemokine may be that one whose expression is induced in the liver following treatment with a DNA damaging agent such as irradiation. Also, the use of a mixture comprising combinations of chemokines e.g. SDF-1, IL-6 and SLF for the enhancement of migration of HSC and/or progenitor cells to the liver is also contemplated according to the inventionton.

The hematopoietic human stem and/or precursor cells to be used according to the invention can be embryonic and/or neonatal such as human cord blood cells and/or adult stem cells (e.g. bone marrow, mobilized peripheral blood cells as described (Kollet et al. 2001). The source of stem and/or precursor cells may be allogeneic (such as HLA-nonmatched donors) preferably syngeneic (such as HLA-matched siblings) and most preferably autologous (i.e. derived from the own patient).

Stem cells and/or progenitor cells can be collected and isolated from peripheral blood of a donor or the patient treated with a mobilization inducing agent such as G-CSF. This agent induces mobilization of such stem cells and/or progenitor cells from hematopoietic organs e.g. bone marrow to the peripheral blood. Further, a chemokine e.g. SDF-1 or a mixture of chemokines could be administered to the patient prior to transplantation of the mobilized HSC/progenitor cells for directing migration of transplanted HSC and/or precursor cells to the liver. In addition or alternatively, the expression of a chemokine or a mixture of chemokines, may be induced in the patient by treatment with a DNA damage agent e.g. ionising irradiation and 5-fluoro uracil. According to the invention, the chemokine or mixture of chemokines will be preferably administered or induced into the patient's liver.

Mobilized stem cells can be injected at an appropriate time before during or after administration of a chemokine or mixture of chemokines and /or agents inducing chemokine expression. In the specific case of autologous transplants, after mobilization of the patient's own stem cells, such cells may not be collected and reinjected, but may be directly induced to migrate to the liver by chemokine administration or by agents inducing chemokine expression prior after or during mobilization.

Preferably, stem cell and/or progenitor cells to be used according to the invention will be obtained by mobilization since mobilization is known to yield more hematopoietic stem cells and progenitor cells than bone marrow surgery.

Cord blood cells can be purchased from a the umbilical cord blood bank at the Coriell Institute for Medical Research (NJ), also own cord blood cells could be used if those cells were cryopreserved after birth.

Hematopoietic stem and progenitor cells are isolated from their cellular mixtures with mature blood cells in said hematopoietic sources by standard techniques (Kollet et al. 2001). E.g. The blood samples are diluted 1:1 in phosphate buffered saline (PBS) without $Mg^{+2}/Ca^{+2}$. Low-density mononuclear cells are collected after standard separation on Ficoll-Paque (Pharmacia Biotech, Uppsala; Sweden) and washed in PBS. $CD34^+$ cells can be purified, using the MACS cell isolation kit and MidiMacs columns (Miltenyi Biotec, Bergisch Gladbach, Germany) according to the manufacturer's instructions, purity of more than 95% can be obtained. Isolated $CD34^+$ cells can be either used immediately for homing experiments or after overnight incubation with RPMI supplemented with 10% fetal calf serum (FCS) or serum free and stem cell factor (SCF) (50 ng/mL). Various techniques can be employed to separate the cells by initially removing cells of dedicated lineage. Antibodies recognising a marker of a specific lineage can be used for separation of the required cells, for example antibodies to the CXCR4 receptor. Also, enriched $CD34^+$ cells can be further labeled with human specific monoclonal antibody (mAb) anti-CD34 FITC (Becton Dickinson, San Jose, CA) and anti-CD38 PE (Coulter, Miami, FL) and sorted for $CD34^+CD38^{-/low}$- or $CD34^+CD38^+$-purified subpopulations by $FACStar^+$ (Becton Dickinson), purity of 97% to 99% may be obtained.

Various techniques of different efficacy can be used to obtain enriched preparations of cells. Such enriched preparations of cells are up to 10%, usually not more than 5%, preferably not more than about 1%, of the total cells present not having the marker can remain with the cell enriched population to be retained.

Procedures for separation of HSC/progenitor cell lineages comprise physical separation e.g. density gradient centrifugation, cell surface (lectin and antibody affinity), magnetic separation etc. A preferred technique that provides good separation is flow cytometry.

Methods of determining the presence or absence of a cell surface marker are well known in the art (Encyclopedia of Immunology Ed. Roitt, Delves, Vol-1 134). Typically, a labelled antibody specific to the marker is used to identify the cell population. Reagents specific for the human cell surface markers Thy-1 and CD34 are known in the art and are commercially available.

Methods for mobilizing stem cells into the peripheral blood are known in the art and generally involve treatment with a chemotherapeutic drug e.g. cydophosphamide (CY) and cytokines e.g. G-CSF, GM-CSF, G-CSF IL3 etc.

Isolated hematopoietic stem cells can be treated ex-vivo prior to transplantation, according to the invention, with growth factors to support survival and growth of homing competent hematopoietic stem cells. In addition the HSC can be co-cultured prior to transplantation with supporting cells such as stromal or feeder layer cells.

The hematopoietic stem cells/progenitor according to the invention can be used in combination with cells from a different type e.g. liver cells.

Genetically modified HSC producing a therapeutic agent may be used according to the method of the invention. Gene transfer to HSC and/or precursors can be carried out by transduction of adeno-associated viruses, retroviruses, lentiviruses and adeno-retroviral chimera, encoding the therapeutic agent e.g. as described by Zheng et al. 2000 and Lotti et al. 2002. Such genetically modified HSC could be used according to the invention in diseases in which liver targeted gene therapy is desired. For example, genetically modified HSC producing the lysosomal enzyme beta glucocerebrosidase could be used according to the invention for the treatment of Gaucher disease or genetically modified HSC producing glucose-6-phosphatase could be used for the treatment in Glycogen storage disease

According to the invention, homing of transplanted or mobilized endogenous HSC to the liver can be achieved by injecting to the liver of a patient in need human SDF-1 and/or other chemokines, preferable from the CXC family. Since DNA-damaging agents such as ionizing irradiation, cyclophosphamide, and 5-fluorouracil, cause an increase in SDF-1 (Ponomaryov et al 2000), such agents can be used in addition to the SDF-1, or chemokine treatment, or as an alternative to SDF-1, or chemokine treatment. Preferably, the agents inducing SDF-1 expression will be administrated directly into the liver. Also in order to increase the SDF-1 concentration in the liver it is possible to irradiate the liver area in a patient in need prior after or during cell transplantation.

Once in the liver hematopoietic stem cells may differentiate into hepatocytes as demonstrated by Lagasse et al. (2000) and Alison et al (2000) and repopulate the liver. Since homing of hematopoietic stem cells (HSC) and/or progenitor cells into the liver is the first step in the initiation of liver repopulation, efficient homing or migration of (HSC) and/or progenitor cells to the liver is crucial for the success of liver repopulation. Therefore, directing migration of HSC and/or progenitor cells, preferably a CD34+ enriched population, more preferably primitive CD34+/CD38-/low cells, to the liver according to the present invention and liver repopulation offers an alternative to liver transplantation.

HSC transplantation according to the invention, may be useful for bridging patients that are waiting to whole organ transplantation, for providing metabolic support during liver failure, and or for replacing whole organ transplantation in metabolic liver diseases.

Recent publications have suggested that adult bone marrow is a potential source of oval cells and hepatocytes. This potential of HSC to generate hepatocytes may have considerable advantages over the use of hepatocytes alone for liver regeneration, e.g. bone marrow can be easily obtained and allows the use of living, related, HLA matched donors and even from the patient's own identical HSC cells. The capacity of HSC to generate liver hepatocytes has been reported in the literature, however with current available protocols in mice, the HSC to hepatocyte transition takes weeks to months. If most of the injected HSC cells reach the bone marrow and only few reach the liver the cells in the bone marrow will engraft and only after engraftment in the bone marrow may reach the liver. Therefore the capability of directing homing of HSC to the liver, in the way described in the present invention, may diminish considerably the time of the transition and make this approach workable.

Homing according to the present invention can be achieved by increasing the concentration of SDF-1 in the liver and/or by treatments which increase the CXCR4 receptor in the membrane of HSC/progenitor cells. Increasing of the CXCR4 receptor in HSC cells may be approached by pre-incubation of the cells with cytokines known to increase expression or the exposure of CXCR4 to the cell surface such as the "IL6RIL6 chimera" protein or the non-fused IL-6 and sIL-6R added separately as described in WO006704. "IL6RIL6 chimera" (also called "IL6RIL6" or IL-6 chimera) is a recombinant glycoprotein obtained fusing the entire coding sequence of the naturally occurring soluble IL-6 Receptor δ-Val to the entire coding sequence of mature naturally occurring IL-6, both from human origin. The IL6RIL6 chimera may be produced in any adequate eukaryotic cells, such as yeast cells, insect cells, and the like. It is preferably produced in mammalian cells, most preferably in genetically engineered CHO cells as described in WO9902552. Whilst the protein from human origin is preferred, it will be appreciated by the person skilled in the art that a similar fusion protein of any other origin may be used according to the invention, as long as it retains the biological activity described herein.

It has been reported that incubation of peripheral blood CD34+ cells on a plastic surface (for about 16 hours) resulted in much larger percentage of CXCR4+ cells and much larger level of CXCR4 expression (Lataillade et al. 2000). Alternatively the HSC can be induced to stably or transiently overexpress CXCR4 and its analogs by introducing expression vectors encoding the CXCR4 gene and analogs. Analogs are defined and prepared similarly to the analogs of SDF-1 as described below. Also a population of HSC enriched with CXCR4, to be used according to the invention, can be isolated by fluorescent activated cell sorting (FACS) as described in WO006704.

The use of a vector for inducing and/or enhancing the endogenous production of CXCR4 is also contemplated according to the invention. The vector may comprise regulatory sequences functional in the cells desired to express CXCR4. Such regulatory sequences may be promoters or enhancers, for example. The regulatory sequence may then be introduced into the right locus of the genome by homologous recombination, thus operably linking the regulatory sequence with the gene, the expression of which is required to be induced or enhanced. This overexpression can be stable or transient. The technology is usually referred to as "endogenous gene activation" (EGA), and it is described e.g. in WO 91/09955.

The present invention concerns an analog of SDF-1, which analog retain essentially the same biological activity of the SDF-1 having essentially only the naturally occurring sequences of SDF-1. Such "analog" may be ones in which up to about 30 amino acid residues may be deleted, added or substituted by others in the SDF-1 protein, such that modifications of this kind do not substantially change the biological activity of the protein analogy with respect to the protein itself.

These analog are prepared by known synthesis and/or by site-directed mutagenesis techniques, or any other known technique suitable therefore.

Any such analog preferably has a sequence of amino acids sufficiently duplicative of that of the basic SDF-1, such as to have substantially similar activity thereto. Thus, it can be determined whether any given analog has substantially the same activity as the basic SDF-1 protein by means of routine experimentation comprising subjecting such an analog to the biological activity tests set forth in the examples below.

Analogs of the SDF-1 protein which can be used in accordance with the present invention, or nucleic acid coding therefore, include a finite set of substantially corresponding sequences as substitution peptides or polynucleotides which can be routinely obtained by one of ordinary skill in the art, without undue experimentation, based on the teachings and guidance presented herein. For a detailed description of protein chemistry and structure, see Schulz, G.E, et al., Principles of Protein Structure, Springer-Verlag, New York, 1978; and Creighton, T.E., Proteins: Structure and Molecular Properties, W.H. Freeman & Co., San Francisco, 1983, which are hereby incorporated by reference. For a presentation of nucleotide sequence substitutions, such as codon preferences, see. See Ausubel et al., Current Protocols in Molecular Biology, Greene Publications and Wiley Interscience, New York, NY, 1987-1995; Sambrook et al., Molecular Cloning: A Laboratory Manual, Cold Spring Harbor Laboratory, Cold Spring Harbor, NY,1989.

Preferred changes for analogs in accordance with the present invention are what are known as "conservative" substitutions. Conservative amino acid substitutions of those in the chimeric protein having essentially the naturally occurring SDF-1 sequences, may include synonymous amino acids within a group which have sufficiently similar physicochemical properties that substitution between members of the group will preserve the biological function of the molecule, Grantham, Science, Vol. 185, pp. 862-864 (1974). It is clear that insertions and deletions of amino acids may also be made in the above -defined sequences without altering their function, particularly if the insertions or deletions only involve a few amino acids, e.g., under thirty, and preferably under ten, and do not remove or displace amino acids which are critical to a functional conformation, e.g., cysteine residues, Anfinsen, "Principles That Govern The Folding of Protein Chains", Science, Vol. 181, pp. 223-230 (1973). Analogs produced by such deletions and/or insertions come within the purview of the present invention.

Preferably, the synonymous amino acid groups are those defined in Table I. More preferably, the synonymous amino acid groups are those defined in Table II; and most preferably the synonymous amino acid groups are those defined in Table III.

TABLE I Preferred Groups of Synonymous Amino Acids

| Amino Acid | Synonymous Group |
|---|---|
| Ser | Ser, Thr, Gly, Asn |
| Arg | Arg, Gln, Lys, Glu, His |
| Leu | Ile, Phe, Tyr, Met, Val, Leu |
| Pro | Gly, Ala, Thr, Pro |
| Thr | Pro, Ser. Ala, Gly, His, Gln, Thr |
| Ala | Gly, Thr, Pro, Ala |
| Val | Met, Tyr, Phe, Ile, Leu, Val |
| Gly | Ala, Thr, Pro, Ser. Gly |
| Ile | Met, Tyr, Phe, Val, Leu, Ile |
| Phe | Trp, Met, Tyr, Ile, Val, Leu, Phe |
| Tyr | Trp, Met, Phe, Ile, Val, Leu, Tyr |
| Cys | Ser, Thr, Cys |
| His | Glu, Lys, Gln, Thr, Arg, His |
| Gln | Glu, Lys, Asn, His, Thr, Arg, Gln |
| Asn | Gln, Asp, Ser, Asn |
| Lys | Glu, Gln, His, Arg, Lys |
| Asp | Glu, Asn, Asp |
| Glu | Asp, Lys, Asn, Gln, His, Arg, Glu |
| Met | Phe, Ile, Val, Leu, Met |
| Trp | Trp |

TABLE II More Preferred Groups of Synonymous Amino Acids

| Amino Acid | Synonymous Group |
|---|---|
| Ser | Ser |
| Arg | His, Lys, Arg |
| Leu | Ile, Phe, Met, Leu |
| Pro | Ala, Pro |
| Thr | Thr |
| Ala | Pro, Ala |
| Val | Met, Ile, Val |
| Gly | Gly |
| Ile | Ile, Met, Phe, Val, Leu |
| Phe | Met, Tyr, Ile, Leu, Phe |
| Tyr | Phe, Tyr |
| Cys | Ser, Cys |
| His | Arg, Gin, His |
| Gln | Glu, His, Gin |
| Asn | Asp, Asn |
| Lys | Arg, Lys |
| Asp | Asn, Asp |
| Glu , | Gln, Glu |
| Met | Phe, Ile, Val, Leu, Met |
| Trp | Trp |

TABLE III Most Preferred Groups of Synonymous Amino Acids

| Amino Acid | Synonymous Group |
|---|---|
| Scr | Ser |
| Arg | Arg |
| Leu | Ile, Met, Leu |
| Pro | Pro |
| Thr | Thr |
| Ala | Ala |
| Val | Val |
| Gly | Gly |
| Ile | Ile, Met, Leu |
| Phe | Phe |
| Tyr | Tyr |
| Cys | Ser, Cys |
| His | His |
| Gln | Gln |
| Asn | Asn |
| Lys | Lys |
| Asp | Asp |
| Glu | Glu |
| Met | Ile, Leu, Met |
| Trp | Trp |

Examples of production of amino acid substitutions in proteins which can be used for obtaining analogs of the protein for use in the present invention include any known method steps; such as presented in US patents RE 33,653, 4,959,314, 4,588,585 and 4,737,462, to Mark et al; 5,116,943 to Koths et al., 4,965,195 to Namen et al; 4,879,111 to Chong et al; and 5,017,691 to Lee et al; and lysine substituted proteins presented in US patent No. 4,904,584 (Straw et al).

In another preferred embodiment of the present invention, any analog of the SDF-1 protein for use in the present invention has an amino acid sequence essentially corresponding to that of the above noted SDF-1 protein of the invention. The term "essentially corresponding to" is intended to comprehend analogs with minor changes to the sequence of the basic chimeric protein which do not affect the basic characteristics thereof, particularly insofar as its ability to SDF-1 is concerned. The type of changes which are generally considered to fall within the "essentially corresponding to" language are those which would result from conventional mutagenesis techniques of the DNA encoding the SDF-1 protein of the invention, resulting in a few minor modifications, and screening for the desired activity in the manner discussed above.

The present invention also encompasses SDF-1 variants. A preferred SDF-1 variant is one having at least 80% amino acid identity, a more preferred SDF-1 variant is one having at least 90% identity and a most preferred variant is one having at least 95% identity to the SDF-1 amino acid sequence.

The term "sequence identity" as used herein means that the amino acid sequences are compared by alignment according to Hanks and Quinn (1991) with a refinement of low homology regions using the Clustal-X program, which is the Windows interface for the Clustal W multiple sequence alignment program (Thompson et al., 1994). The Clustal-X program is available over the internet at ftp://ftp-igbmc.u-strasbg.fr/pub/clustalx/. Of course, it should be understood that if this link becomes inactive, those of ordinary skill in the art can find versions of this program at other links using standard internet search techniques without undue experimentation. Unless otherwise specified, the most recent version of any program referred herein, as of the effective filing date of the present application, is the one which is used in order to practice the present invention.

If the above method for determining "sequence identity" is considered to be non-enabled for any reason, then one may determine sequence identity by the following technique. The sequences are aligned using Version 9 of the Genetic Computing Group's GDAP (global alignment program), using the default (BLOSUM62) matrix (values -4 to +11) with a gap open penalty of -12 (for the first null of a gap) and a gap extension penalty of -4 (per each additional consecutive null in the gap). After alignment, percentage identity is calculated by expressing the number of matches as a percentage of the number of amino acids in the claimed sequence.

Analogs in accordance with the present invention include those encoded by a nucleic acid, such as DNA or RNA, which hybridizes to DNA or RNA under stringent conditions and which encodes a SDF-1 protein in accordance with the present invention, comprising essentially all of the naturally-occurring sequences encoding SDF-1. For example, such a hybridising DNA or RNA maybe one encoding the same protein which nucleotide differs in its nucleotide sequence from the naturally-derived nucleotide sequence by virtue of the degeneracy of the genetic code, i.e., a somewhat different nucleic acid sequence may still code for the same amino acid sequence, due to this degeneracy. Further, as also noted above, the amount of amino acid changes (deletions, additions, substitutions) is limited to up to about 30 amino acids.

The term "hybridization" as used herein shall include any process by which a strand of nucleic acid joins with complementary strand through a base pairing (Coombs J, 1994, Dictionary of Biotechnology, stokton Press, New York NY). "Amplification" is defined as the production of additional copies of a nucleic acid sequence and is generally carried out using polymerase chain reaction technologies well known in the art (Dieffenbach and Dveksler, 1995, PCR Primer, a Laboratory Manual, Cold Spring Harbor Press, Plainview NY).

"Stringency" typically occurs in a range from about Tm-5°C (5°C below the melting temperature of the probe) to about 20°C to 25°C below Tm.

The term "stringent conditions" refers to hybridization and subsequent washing conditions which those of ordinary skill in the art conventionally refer to as "stringent". See Ausubel et al., Current Protocols in Molecular Biology, Greene Publications and Wiley Interscience, New York, NY, 1987-1995; Sambrook et al., Molecular Cloning: A Laboratory Manual, Cold Spring Harbor Laboratory, Cold Spring Harbor, NY, 1989.

As used herein, stringency conditions are a function of the temperature used in the hybridization experiment, the molarity of the monovalent cations and the percentage of formamide in the hybridization solution. To determine the degree of stringency involved with any given set of conditions, one first uses the equation of Meinkoth et al. (1984) for determining the stability of hybrids of 100% identity expressed as melting temperature Tm of the DNA-DNA hybrid:

$$Tm = 81.5\ C + 16.6\ (LogM) + 0.41\ (\%GC) - 0.61\ (\%\ form) - 500/L$$

where M is the molarity of monovalent cations, %GC is the percentage of G and C nucleotides in the DNA, % form is the percentage of formamide in the hybridization solution, and L is the length of the hybrid in base pairs. For each 1 C that the Tm is reduced from that calculated for a 100% identity hybrid, the amount of mismatch permitted is increased by about 1%. Thus, if the Tm used for any given hybridization experiment at the specified salt and formamide concentrations is 10 C below the Tm calculated for a 100% hybrid according to the equation of Meinkoth, hybridization will occur even if there is up to about 10% mismatch.

As used herein, "highly stringent conditions" are those which provide a Tm which is not more than 10 C below the Tm that would exist for a perfect duplex with the target sequence, either as calculated by the above formula or as actually measured. "Moderately stringent conditions" are those, which provide a Tm, which is not more than 20 C below the Tm that would exist for a perfect duplex with the target sequence, either as calculated by the above formula or as actually measured. Without limitation, examples of highly stringent (5-10 C below the calculated or measured Tm of the hybrid) and moderately stringent (15-20 C below the calculated or measured Tm of the hybrid) conditions use a wash solution of 2 X SSC (standard saline citrate) and 0.5% SDS (sodium dodecyl sulphate) at the appropriate temperature below the calculated Tm of the hybrid. The ultimate stringency of the conditions is primarily due to the washing conditions, particularly if the hybridization conditions used are those, which allow less stable hybrids to form along with stable hybrids. The wash conditions at, higher stringency then remove the less stable hybrids. A common hybridization condition that can be used with the highly stringent to moderately stringent wash conditions described above is hybridization in a solution of 6 X SSC (or 6 X SSPE (standard saline-phosphate-EDTA)), 5 X Denhardt's reagent, 0.5% SDS, 100 microgram/ml denatured, fragmented salmon sperm DNA at a temperature approximately 20 to 25 C below the Tm. If mixed probes are used, it is preferable to use tetramethyl ammonium chloride (TMAC) instead of SSC (Ausubel, 1987, 1999).

The term "fused protein" refers to a polypeptide comprising an SDF-1, or a analogues or fragment thereof, fused with another protein, which, e.g., has an extended residence time in body fluids. An SDF-1 may thus be fused to another protein, polypeptide or the like, e.g., an immunoglobulin or a fragment thereof.

"Functional derivatives" as used herein cover derivatives of SDF-1 and their analogues and fused proteins, which may be prepared from the functional groups which occur as side chains on the residues or the N- or C-terminal groups, by means known in the art, and are included in the invention as long as they remain pharmaceutically acceptable, i.e. they do not destroy the activity of the protein which is substantially similar to the activity of SDF-1 and do not confer toxic properties on compositions containing it.

These derivatives may, for example, include polyethylene glycol side-chains, which may mask antigenic sites and extend the residence of an SDF-1 in body fluids. Other derivatives include aliphatic esters of the carboxyl groups, amides of the carboxyl groups by reaction with ammonia or with primary or secondary amines, N-acyl derivatives of free amino groups of the amino acid residues formed with acyl moieties (e.g. alkanoyl or carbocyclic aroyl groups) or O-acyl derivatives of free hydroxyl groups (for example that of seryl or threonyl residues) formed with acyl moieties.

As "Fragment" of an SDF-1 , analogue and fused proteins, the present invention covers any fragment or precursors of the polypeptide chain of the protein molecule alone or together with associated molecules or residues linked thereto, e.g., sugar or phosphate residues, or aggregates of the protein molecule or the sugar residues by themselves, provided said fraction has substantially similar activity to SDF-1.

A chemokine e.g. SDF-1 alone or a combination of chemokines such as IL-6, SDF-1 and SLF could be used to support migration of hematopoietic stem cells/precursor to the liver of a patient in need. Also, A chemokine e.g. SDF-1 alone or a combination of chemokines such as IL-6, SDF-1 and SLF could be administrated to a patient prior during or after HSC and/or progenitor transplantation and/or mobilization, wherein the transplantation is autologous or heterologous.

A chemokine e.g. SDF-1 alone or in combination with other chemokines could be used according to the invention in patients for whom liver transplantation therapy is indicated, while waiting for a matching donor or as an alternative for liver transplantation. A chemokine e.g. SDF-1 alone or in combination with other chemokines could be used according to the invention in patients who rejected liver transplants.

A chemokine e.g. SDF-1 alone or in combination with other chemokines could be used according to the invention in patients for whom gene therapy is indicated. A chemokine e.g. SDF-1 alone or in combination with other chemokines could be administrated to a patient prior during or after genetically modified HSC and/or progenitor transplantation wherein the HSC and/or progenitor cells are autologous or heterologous.

The method of the invention comprising enhancement of HSC and/or progenitor cell migration to the liver according to the invention may be beneficial for a subject suffering from a liver disease. Liver disease has numerous causes. Hepatitis involves inflammation and damage to the hepatocytes, which may be a result of infectious, toxic or immunologic agents. Hepatitis A, B, and C are caused by viruses. Alcohol abuse and chronic use of drugs and can cause liver damage. The liver may be affected by autoimmune disorders for example rheumatic diseases, Lupus erythromatosus and rheumatoid arthritis, inflammatory bowel disease such as ulcerative colitis and Crohn's disease.

The present invention also relates to pharmaceutical compositions prepared for administration of a chemokine e.g. SDF-1 or an analogue, fused protein, functional derivative and/or fragment thereof , or a mixture of chemokines by mixing the chemokine/s , with physiologically acceptable carriers, and/or stabilizers and/or excipients, and prepared in dosage form, e.g., by lyophilization in dosage vials.

The invention further relates to pharmaceutical compositions, particularly useful for enhancing homing of HSC and/or progenitors to the liver, which comprise a therapeutically effective amount of SDF-1 and/or a therapeutically effective amount of a different chemokine and/or a pharmaceutically effective amount of a mixture of chemokines.

The present invention further relates to pharmaceutical compositions comprising a pharmaceutically acceptable carrier and a chemokine e.g. SDF-1 or an analogue, fused protein, functional derivative and/or fragment thereof or a mixture of chemokines for the treatment of liver diseases. Preferably the SDF-1 may be administered by direct injecting into the hepatic parenchyma before after or during cell transplantation and/or mobilization. Alternatively SDF-1 may be induced preferable by the local administration of DNA damaging agents such as by irradiation and/ or chemotherapy.

SDF-1 or an analogue fused protein, functional derivative and/or fragment thereof, as described above are the preferred active ingredients of the pharmaceutical compositions.

The pharmaceutical compositions may comprise a pharmaceutically acceptable carrier, a chemokine e.g. SDF-1 or its analogues, fusion proteins, functional derivative or fragment thereof and optionally further including one or more chemokine.

The definition of "pharmaceutically acceptable" is meant to encompass any carrier, which does not interfere with effectiveness of the biological activity of the active ingredient and that is not toxic to the host to which it is administered. For example, for parenteral administration, the active protein(s) may be formulated in a unit dosage form for injection in vehicles such as saline, dextrose solution, serum albumin and Ringer's solution.

The active ingredients of the pharmaceutical composition according to the invention can be administered to an individual in a variety of ways. A therapeutically efficacious route of administration can be used, for example absorption through epithelial or endothelial tissues or by gene therapy wherein a DNA molecule encoding the active agent is administered to the patient (e.g. via a vector) which causes the active agent to be expressed and secreted in vivo. In addition, the protein(s) according to the invention can be administered together with other components of biologically active agents such as pharmaceutically acceptable surfactants, excipients, carriers, diluents and vehicles.

For parenteral (e.g. intravenous, intramuscular) administration, the active protein(s) can be formulated as a solution, suspension, emulsion or lyophilized powder in association with a pharmaceutically acceptable parenteral vehicle (e.g. water, saline, dextrose solution) and additives that maintain isotonicity (e.g. mannitol) or chemical stability (e.g. preservatives and buffers). The formulation is sterilized by commonly used techniques.

The bioavailability of the active protein(s) according to the invention can also be ameliorated by using conjugation procedures which increase the half-life of the molecule in the human body, for example linking the molecule to polyethylenglycol, as described in the PCT Patent Application WO 92/13095.

The therapeutically effective amounts of the active protein(s) will be a function of many variables, including the type of chemokine used, any residual cytotoxic activity exhibited by the chemokine, the route of administration, the clinical condition of the patient.

A "therapeutically effective amount" is such that when administered, the chemokine results in enhanced migration of HSC to the liver. The dosage administered, as single or multiple doses, to an individual will vary depending upon a variety of factors, including the chemokine pharmacokinetic properties, the route of administration, patient conditions and characteristics (sex, age, body weight, health, size), extent of symptoms, concurrent treatments, frequency of treatment and the effect desired. Adjustment and manipulation of established dosage ranges are well within the ability of those skilled in the art, as well as in vitro and in vivo methods of determining the effect of the chemokine in an individual.

The route of administration which is preferred according to the invention is administration by direct injecting into the hepatic parenchyma.

According to the invention, the chemokine e.g. SDF-1 can be administered to an individual prior to, simultaneously or sequentially with other therapeutic regimens (e.g. multiple drug regimens) or agents, in a therapeutically effective amount, in particular with transplanted HSC and/or progenitor cells, and/or mobilization agents and/or DNA damaging agents.

The invention further relates to a method of treatment of liver disease, comprising administering a pharmaceutically effective amount of a chemokine to a patient in need thereof.

While embodiments 1 to 32 are defined in the accompanying claims, further embodiments of the invention are described below.

Embodiment 33 relates to a method for increasing homing of hematopoietic stem cells (HSC) and/or hematopoietic progenitor cells (HPC) to the liver of a subject in need comprising: administering and/or mobilizing said cells and treating the subject in need with one or more chemokines.

Embodiment 34 relates to a method according to embodiment 33, wherein the chemokine belongs to the CXC chemokine family.

Embodiment 35 relates to a method according to embodiments 33 or 34, wherein the chemokine is SDF-1 or an analog fusion protein variant, functional derivative, or fragment thereof.

Embodiment 36 relates to a method according to anyone of embodiments 33 to 35, wherein the chemokine is injected into the liver of a subject in need.

Embodiment 37 relates to a method according to anyone of embodiments 33 to 36, wherein the chemokine is induced by treatment with DNA damaging agents.

Embodiment 38 relates to a method according to embodiment 37, wherein the chemokine is induced by irradiation.

Embodiment 39 relates to a method according to embodiment 37, wherein the chemokine is induced by cyclophosphamide.

Embodiment 40 relates to a method according to embodiment 37, wherein the chemokine is induced by 5-fluoroacil.

Embodiment 41 relates to a method according to anyone of embodiments 33 to 40, wherein the administered HSC/HPC are of embryonic origin.

Embodiment 42 relates to a method according to anyone of embodiments 33 to 40, wherein the administered HSC/HPC are of neonatal origin.

Embodiment 43 relates to a method according to embodiment 42, wherein the administered HSC/HPC are from human umbilical cord blood.

Embodiment 44 relates to a method according to anyone of embodiments 33 to 40, wherein the administered HSC/HPC are of adult origin.

Embodiment 45 relates to a method according to embodiment 44, wherein the administered HSC/HPC are from the bone marrow.

Embodiment 46 relates to a method according to embodiment 44, wherein the administered HSC/HPC are from mobilized peripheral blood.

Embodiment 47 relates to a method according to anyone of embodiments 41 to 46, wherein the administered HSC/HPC are allogeneic.

Embodiment 48 relates to a method according to anyone of embodiments 41 to 46, wherein the administered HSC/HPC are syngeneic.

Embodiment 49 relates to a method according to anyone of embodiments 42 to 46, wherein the administered HSC/HPC are autologous cells.

Embodiment 50 relates to a method according to embodiment 49, further comprising the administration of a mobilizing agent selected from IL-3, SLF, GM-CSF and G-CSF.

Embodiment 51 relates to a method according to embodiment 50, wherein the mobilizing agent comprises G-CSF.

Embodiment 52 relates to a method according to embodiments 50 or 51, wherein the mobilizing agent is administrated prior to the chemokine treatment.

Embodiment 53 relates to a method according to anyone of embodiments 50 to 52, wherein the mobilized HSC/HPC are collected from, and administered into the subject in need.

Embodiment 54 relates to a method according to anyone of embodiments 41 to 53, wherein the administered HSC/HPC are CD34+ cells.

Embodiment 55 relates to a method according to embodiment 54, wherein the administered HSC/HPC are CD34+/CD38-/low cells.

Embodiment 56 relates to a method according to anyone of embodiments 33 to 55, wherein the administered HSC/HPC are genetically modified cells producing a therapeutic agent.

Embodiment 57 relates to a method according to anyone of embodiments 41 to 56, wherein the administered HSC/HPC were treated prior transplantation with a growth factor.

Embodiment 58 relates to a method according to embodiment 57, wherein the factor is IL-6.

Embodiment 59 relates to a method according to embodiment 57, wherein the factor is IL-6 and IL-6R.

Embodiment 60 relates to a method according to embodiment 57 wherein the factor is sIL6R/IL6 chimeric protein.

Embodiment 61 relates to a method according to embodiment 57, wherein the factor is SFL.

Embodiment 62 relates to a method according to anyone of embodiments 41 to 61, wherein the administered HSC/HPC were treated prior transplantation with supporting cells.

Embodiment 63 relates to a method according to anyone of embodiments 41 to 57, further comprising administration of cells from a different type.

Embodiment 64 relates to a method according to embodiment 63, wherein the cells of different type are hepatic cells.

Embodiment 65 relates to a method according to anyone of embodiments 49 to 52, wherein mobilized HSC/HPC are not collected and are allowed to migrate directly to the liver of the subject in need.

Embodiment 66 relates to a method according to anyone of embodiments 33 to 65, wherein the subject in need suffers from a liver disease.

Embodiment 67 relates to a method according to anyone of embodiments 33 to 66, wherein the subject needs liver targeted gene therapy.

Embodiment 68 relates to a method according to embodiment 67, wherein the subject suffers from Gaucher disease.

Embodiment 69 relates to a method according to embodiment 67, wherein the subject suffers from Glycogen storage disease.

Having now fully described this invention, it will be appreciated by those skilled in the art that the same can be performed within a wide range of equivalent parameters, concentrations and conditions without departing from the spirit and scope of the invention and without undue experimentation.

While this invention has been described in connection with specific embodiments thereof, it will be understood that it is capable of further modifications. This application is intended to cover any variations, uses or adaptations of the invention following, in general, the principles of the invention and including such departures from the present disclosure as come within known or customary practice within the art to which the invention pertains and as may be applied to the essential features hereinbefore set forth as follows in the scope of the appended claims.

All references cited herein, including journal articles or abstracts, published or unpublished U.S. or foreign patent application, issued U.S. or foreign patents or any other references, are entirely incorporated by reference herein, including all data, tables, figures and text presented in the cited references. Additionally, the entire contents of the references cited within the references cited herein are also entirely incorporated by reference.

Reference to known method steps, conventional methods steps, known methods or conventional methods is not any way an admission that any aspect, description or embodiment of the present invention is disclosed, taught or suggested in the relevant art.

The foregoing description of the specific embodiments will so fully reveal the general nature of the invention that others can, by applying knowledge within the skill of the art (including the contents of the references cited herein), readily modify and/or adapt for various application such specific embodiments, without undue experimentation, without departing from the general concept of the present invention. Therefore, such adaptations and modifications are intended to be within the meaning an range of equivalents of the disclosed embodiments, based on the teaching and guidance presented herein. It is to be understood that the phraseology or terminology herein is for the purpose of description and not of limitation, such that the terminology or phraseology of the present specification is to be interpreted by the skilled artisan in light of the teachings and guidance presented herein, in combination with the knowledge of one of ordinary skill in the art.

EXAMPLES

**Example 1.**

**Homing of transplanted human hematopoietic stem cells to the liver.**

It has been reported that hematopoietic stem cells from donor rats can reach to the liver of recipient rats and regenerate into hepatic cells (Petersen et al. 1999). However, whether they reach the liver directly or via a hematopoietic organ such as the bone marrow or spleen is unknown. To clarify this point, CD34+ enriched human stem cells (05-1x $10^6$) from mobilized peripheral blood (MPB) of a healthy donor were transplanted into NOD/SCID mice (Peled et al. 1999 and Kollet et al 2001) and shortly after transplantation (16 hours) mice were sacrificed and the presence of human cells in the liver was monitored.

To get enriched CD34+ cells (enrichment of 80% and above), human mobilized peripheral blood (from healthy donors treated with GCSF) was subjected to fractionation of low-density mononuclear cells (NMC) on Ficoll-Paque (Pharmacia Biotech, Uppsala, Sweden) followed by a mini MACS kit (Miltney Biotec, Bergisch Gladbach, Germany).

Human enriched CD34+ mobilized peripheral blood (MPB) cells were incubated and co-transplanted with anti CXCR4 antibody (12G5, 10μg/mouse) or with media alone (control). 0.5-1x106 CD34+ MPB cells were transplanted into NOD/SCID mice by intravenous (IV) injection, 24 hours post 375cGy sublethally irradiation. Mice were sacrificed 16h later. Single cell suspension were prepared and introduced to flow cytometry (FACS) analysis, using anti human CD34 FITC and anti CD38 PE (figure 1).

The results obtained show that human stem cells and progenitor cells home to the bone marrow and spleen and that this homing, as previously reported, is dependent on SDF-1/CXCR4, since pre-incubation (30 minutes) and co-transplantation with the anti- CXCR4 antibody greatly inhibits homing to the bone marrow and spleen.

A comparable number of human stem cells and progenitor cells were found also in the liver of these mice. When the CD34+ enriched stem cells were pre treated and co-transplanted with anti CXCR4 neutralizing antibody, homing of human CD34+ enriched stem cells to the liver was significantly reduced, indicating that similarly to bone marrow and spleen, homing to the liver is dependent on SDF-1 signalling via CXCR4.

These results show for the first time that hematopoietic stem cells and progenitor cells can home directly to the liver and that this homing requires CXCR4/SDF-1 interaction.

**Example 2.**

**SDF-1 mediated homing of HSC to the liver**

SDF-1 is highly expressed by human bone marrow osteoblast and endothelial cells.

Clinical bone marrow transplantation requires conditioning of the recipient with radiation or chemotherapy before stem cell transplantation. It has been reported that conditioning mice with DNA-damaging agents such as ionizing irradiation, cyclophosphamide, and 5-fluorouracil, causes an increase in SDF-1 expression and in CXCR4-dependent homing and repopulation of bone marrow by human stem cells transplanted into NOD/SCID mice (Ponomaryov et al 2000). Since migration of human progenitors to the murine liver requires signaling trough CXCR4 (see finding in previous example), the level of SDF-1 was measured in the liver of irradiated mice and compared to the level of SDF-1 in non irradiated mice. A significant increase in SDF-1 expression was found following total body irradiation in the liver (not shown).

The following experiment was carried out in order to test whether homing of human CD34+ enriched MPB cells to the liver of NOD/ SCID mice requires high concentrations of human SDF-1 in the liver.

Frozen mouse mobilized peripheral blood (MPB) CD34+ cells were thawed, and incubated at 37°C over night with 50ng/ml SLF for recovery. In contrast to the experiment described in example 1, this experiment was carried out with non irradiated mice (not pre-conditioned) and also in non-irradiated mice in which human SDF-1 has been injected directly into the liver immediately prior transplantation.

Briefly, non-irradiated NOD/SCID mice were anaesthetized and 1 microgram SDF-1 (in 50 microliter PBS) were injected into the right lobe of the liver. Next, treated and control mice were transplanted intravenously with 8x$10^5$ CD34+ MPB enriched cells. In one group , CD34+ cells were incubated for 30 min. (4°C) with 10 micrograms of neutralizing anti-human CXCR4 mAb (12G5), and co-transplanted without washing. Four hours later, the injected lobe was harvested, a single cell suspension was prepared and introduced to flow cytometry analysis, using human specific anti CD34-FITC and anti CD38-PE mAb.Injection of SDF-1 to the liver creates a positive gradient in the liver and a negative gradient in the blood. Since this gradient is maintained for about 4 hours only, therefore the mice were sacrificed and homing was tested rapidly, four hours after cell injection. The results summarized in Table 1 below show that homing to the liver was very low in non-irradiated mice. In contrast, a considerable number of human stem cells and progenitor cells were observed in non-irradiated mice in which human SDF-1 was injected into the liver. Co-transplantation of stem cells with anti CXCR4 (12G5) antibodies inhibited homing in human SDF-1 treated mice, indicating that homing to the liver is specifically induced by human SDF-1 and its interaction with CXCR4.

**Table 1**

| Treatment | - | SDF-1 | SDF-1 + 12G5 |
|---|---|---|---|
| Number of human cells in the liver per 1X10$^6$ total aquired cells | 11 | 38 | 13 |
| % | 29.0 | 100 | 34.2 |

**Example 3:**

**Effect of SLF and sIL6R/IL6 Chimera in homing of hematopoietic human cells to the liver**

Stem-cell factor (SLF, steel factor or ckit-ligand) has been found to be important for survival and proliferation of the most primitive pluripotential hematopoietic stem cells capable of long-term engraftment in recipient bone marrow (McKenna et al, 1995). SLF and IL-6 are known to increase surface CXCR4 expression in CD34+ cell and their migration toward SDF-1 gradients. The sIL6R/IL6 chimeric protein (comprising the soluble IL-6R linked to IL-6) can act also in a more primitive stem cell population within the CD34+ stem cells, which lacks the IL-6 receptor but has the GP130 receptor. To study the effect of SLF and/or sIL6R/IL6 in homing of hematopoietic stem cells and progenitor cells, NOD/SCID mice (Peled et al. 1999 and Kollet et al 2001 and example 1) are subjected to sub-lethal irradiation and injected into the tail vein with 05- 1x10$^6$ human CD34+ enriched MPB that are maintained with SLF (50ng/ml) and/or sIL6R/IL6 (100ng/ml) for 3 days in liquid culture prior transplantation. After 16 hours, the mice are sacrificed and the liver is taken to monitor the presence of human CD34+ cells. Homing of human cells to the liver of mice is evaluated by FACS analysis of CD34+ labelled cells (see example 1).
Mice, which were injected with cells treated with the cytokines, will show increased homing of human CD34+ to the liver.
Thus increasing the cell surface CXCR4 on hematopoietic stem cells and progenitor cells may support migration to the liver.

**REFERENCES**

Alison et al. 2000 "Hepatocytes from non-hepatic adult stem cells" Nature 406, 257.

Bleul CC, Fuhlbrigge RC, Casasnovas JM, Aiuti A, Springer TA. 1996 "A highly efficacious lymphocyte chemoattractant, stromal cell-derived factor 1 (SDF-1)" J Exp Med 184, 1101-1109.

Diehl A M; Rai R M Liver regeneration 3: Regulation of signal transduction during liver regeneration.FASEB 1996, 10 (2) p215-27.

Drize N, Chertkov J, Samoilina N, Zander A. 1996 "Effect of cytokine treatment (granulocyte colony-stimulating factor and stem cell factor) on hematopoiesis and the circulating pool of hematopoietic stem cells in mice." Exp Hematol 24, 816-822.

Duhrsen U, Villeval JL, Boyd J, Kannourakis G, Morstyn G, Metcalf D.1988 "Effects of recombinant human granulocyte colony-stimulating factor on hematopoietic progenitor cells in cancer patients." Blood 72,2074-2081.

Grisham and Thorgeirsson, in Stem Cells, C.S. Ed. (Academic Press, San Diego, CA, 1997), chap, 8.

Kim and Broxmeyer 1998 "In vitro behavior of hematopoietic progenitor cells under the influence of chemoattractants: stromal cell-derived factor-1, steel factor, and the bone marrow environment." blood,1,100-110.

Kim CH, Broxmeyer HE. SLC/exodus2/6Ckine/TCA4 induces chemotaxis of hematopoietic progenitor cells: differential activity of ligands of CCR7, CXCR3, or CXCR4 in chemotaxis vs. suppression of progenitor proliferation. J Leuk Biol 1999; 66:455

Kollet 0, Spiegel A, Peled A, Petit I, Byk T, Hershkoviz R, Guetta E, Barkai G, Nagler A, Lapidot T "Rapid and efficient homing of human CD34(+)CD38(-/low)CXCR4(+) stem and progenitor cells to the bone marrow and spleen of NOD/SCID and NOD/SCID/B2m(null) mice" 2001 Blood 97,3283-91.

Lagasse et al. 2000 "Purified hematopoietic stem cells can differentiate into hepatocytes in vivo." Nature medicine 6,1229-34.

Lagasse et al. 20001 "Toward regenerative medicine." Immunity 14,425-36. Lapidot 2001 Ann. NY Acad. Sci. "Mechanism of human stem cell migration and repopulation of NOD/SCID and B2mnull NOD/SCID mice. The role of SDF-1/CXCR4 interactions" 938 83-95

Lotti et al. Journal of Virology. 2002 "Transcriptional targeting of lentiviral vectors by long terminal repeat enhancer replacement." 76 (8) 3996-4007.

Mazo IB, von Andrian UH. 1999 "Adhesion and homing of blood-borne cells in bone marrow microvessels." Journal of leukocyte Biology 66,25-32.

Novelli, M. et al. 1996 "Polyclonal origin of colonic adenomas in an XO/XY patient with FAP" Science 272, 1187-1190.

Novikoff PM, Yam A, Oikawa I. "Blast-like cell compartment in carcinogen-induced proliferating bile ductule. "Am J Pathol 1996 May;148(5):1473-92.

Papayannopoulou T 1999 "Hematopoietic stem/progenitor cell mobilization. A continuing quest for etiologic mechanisms." Ann N Y Acad Sci 872,187-197; discussion 197-9.

Peled A, Petit I, Kollet 0, Magid M, Ponomaryov T, Byk T, Nagler A, Ben-Hur H, Many A, Shultz L, Lider O, Alon R, Zipori D, Lapidot T.1999 "Dependence of human stem cell engraftment and repopulation of NOD/SCID mice on CXCR4." Science 283, 845-848.

Peled A, Grabovsky V, Habler L, Sandbank J, Arenzana-Seisdedos F, Petit I, Ben-Hur H, Lapidot T, Alon R 1999 "The chemokine SDF-1 stimulates integrin-mediated arrest of CD34(+) cells on vascular endothelium under shear flow." The Journal of Clinical Investigation, 104,1199-1211.

Petersen et al. 1999 "Bone marrow as a potential source of hepatic oval cells" SCIENCE 284, 1168-70.

Ponomaryov T, Peled A, Petit I, et al. "Induction of the chemokine stromal-derived factor-1 following DNA damage improves human stem cell function." J Clin Invest. 2000;106:1331-1339

Rosu-Myles M, Gallacher L, Murdoch B, Hess DA, Keeney M, Kelvin D, Dale L, Ferguson SS, Wu D, Fellows F, Bhatia M. 2000 "The human hematopoietic stem cell compartment is heterogeneous for CXCR4 expression." PNAS 97,14626-14631.

Siena S, Bregni M, Brando B, Ravagnani F, Bonadonna G, Gianni AM., 1989" Circulation of CD34+ hematopoietic stem cells in the peripheral blood of high-dose cyclophosphamide-treated patients: enhancement by intravenous recombinant human granulocyte-macrophage colony-stimulating factor." Blood 74,1905-1914.

Strom SC, Fisher RA, Thompson MT, Sanyal AJ, Cole PE, Ham JM, Posner MP. Hepatocyte transplantation as a bridge to orthotopic liver transplantation in terminal liver failure. Transplantation 1997 Feb 27;63(4):559-69.

Suzuki et al. Intern Immunol. 1998 "Loss of SDF-1 receptor expression during positive selection in the thymus" 10 8 1049-1056.

Sweeny, E.A., Priestley, G., Nakamoto, B., Papayannopoulou, T. 2000 "Sulfated Polysaccharides Increase Plevels of SDF-1 in Monkeys and Mice: Involvement in Mobilization of Stem/Progenitor Cells." Abstracts of the 42nd annual meeting of the American society of Haematology, December 1-5.

Tanaka et al. 1999 "Fetal microchimerism alone does not contribute to the induction of primary biliary cirrhosis" Hepatology 30, 833-838.

Van Eyken et a]. 1993 "Cytokeratins and the liver." Liver 13,113-22.

Weglarz TC, Degen JL and Sandgren EP. Hepatocyte transplantation into diseased mouse liver. 2000 Dec;Kinetics of parenchymal repopulation and identification of the proliferative capacity of tetraploid and octaploid hepatocytes. Am J Pathol. 157(6):1963-74.

Wolfe et al.1985 J Mol Biol. "Isolation and characterization of an alphoid centromeric repeat family from the human Y chromosome." 182,477-485.

Zheng et al Nat Biotechnology 2000 "Genomic integration and gene expression by a modified adenoviral vector." 18,176-180.

**Claims**

1. The use of one or more chemokines and/or a DNA damaging agent inducing said chemokine/s in the manufacture of a medicament for enhancing homing of hematopoietic stem cells (HSC) and/or hematopoietic progenitor cells (HPC) to the liver of a subject in need.

2. The use according to claim 1, wherein the chemokine belongs to the CXC chemokine family.

3. The use according to anyone of claims 1 to 2, wherein the chemokine is SDF-1 or an analog, fusion protein, functional derivatives, variant or fragment thereof.

4. The use according to anyone of claims 1 to 3, wherein the damaging agent is cyclophosphamide.

5. The use according to anyone of claims 1 to 3, wherein the damaging agent is 5-fluoroacil.

6. The use according to anyone of claims 1 to 3, wherein the damaging agent is irradiation.

7. The use according to anyone of the preceding claims, wherein said HSC/HPC are of embryonic origin.

8. The use according to anyone of claims 1 to 6, wherein said HSC/HPC are from neonatal origin.

9. The use according to claim 8, wherein said HSC/HPC are from umbilical cord blood.

10. The use according to anyone of claims 1 to 6, wherein said HSC/HPC are of adult origin.

11. The use according to claim 10, wherein said HSC/HPC are from the bone marrow.

12. The use according to claim 10, wherein the said HSC/HPC are from mobilized peripheral blood.

13. The use according to anyone of claims 7 to 12, wherein said HSC/HPC are allogeneic cells.

14. The use according to anyone of claims 7 to 12, wherein said HSC/HPC are syngeneic cells.

15. The use according to anyone of claims 8 to 12, wherein said HSC/HPC are autologous cells.

16. The use according to claim 15, wherein the medicament further comprises a mobilizing agent selected from IL-3, SLF, GM-CSF and G-CSF.

17. The use according to claim 16, wherein the mobilizing agent comprises G-CSF.

18. The use according to anyone of claims 1 to 17, wherein said HSC/HPC are CD34+ cells.

19. The use according to claim 18, wherein said HSC/HPC are CD34+/CD38-/low.

20. The use according to anyone to claims 1 to 19, wherein said HSC/HPC are genetically modified cells producing a therapeutic agent.

21. The use according to anyone of claims 1 to 20, wherein said HSC/HPC were treated with a growth factor.

22. The use according to claim 21, wherein the factor is IL-6.

23. The use according to claim 21, wherein the factor is IL-6 and IL-6R.

24. The use according to claim 21, wherein the factor is sIL6R/IL6 chimeric protein.

25. The use according to claim 21, wherein the factor is SFL.

26. The use according to anyone of the previous claims, wherein said HSC/HPC were treated with supporting cells.

27. The use according to anyone of the previous claims, wherein the medicament further comprises a cell type which is different from said HSC/HPC.

28. The use according to claim 27, where the cell is of hepatic type.

29. The use according to anyone of the previous claims, wherein the subject in need suffers from a liver disease.

30. The use according to anyone of claims 1 to 29, wherein the subject needs liver targeted gene therapy.

31. The use according to claim 30, wherein the subject suffers from Gaucher disease.

32. The use according to claim 30, wherein the subject suffers from glycogen storage disease.

Figure 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5087570 A
- US 5061620 A
- WO 0171016 A, Lagasse
- WO 006704 A
- WO 9902552 A
- WO 9109955 A
- US RE33653 E
- US 4959314 A

- US 4588585 A
- US 4737462 A, Mark
- US 5116943 A, Koths
- US 4965195 A, Namen
- US 4879111 A, Chong
- US 5017691 A, Lee
- US 4904584 A, Straw
- WO 9213095 A


**Non-patent literature cited in the description**

- **BLAU HM.** *Cell,* 2001, vol. 105, 829
- **BRYON E.** *Blood Cells, Molecules, and Diseases,* 2001, vol. 27, 590
- **WEBBER EM.** *Hepatology,* 1998, vol. 28, 1226
- *Encyclopedia of Immunology,* vol. 1, 134
- **SCHULZ, G.E et al.** Principles of Protein Structure. Springer-Verlag, 1978
- **CREIGHTON, T.E.** Proteins: Structure and Molecular Properties. W.H. Freeman & Co, 1983
- **AUSUBEL et al.** Current Protocols in Molecular Biology. Greene Publications and Wiley Interscience, 1987
- **SAMBROOK et al.** Molecular Cloning: A Laboratory Manual. Cold Spring Harbor Laboratory, 1989
- **GRANTHAM.** *Science,* 1974, vol. 185, 862-864
- **ANFINSEN.** Principles That Govern The Folding of Protein Chains. *Science,* 1973, vol. 181, 223-230
- **COOMBS J.** Dictionary of Biotechnology. stokton Press, 1994
- **DIEFFENBACH ; DVEKSLER.** PCR Primer, a Laboratory Manual. Cold Spring Harbor Press, 1995
- **ALISON et al.** Hepatocytes from non-hepatic adult stem cells. *Nature,* 2000, vol. 406, 257
- **BLEUL CC ; FUHLBRIGGE RC ; CASASNOVAS JM ; AIUTI A ; SPRINGER TA.** A highly efficacious lymphocyte chemoattractant, stromal cell-derived factor 1 (SDF-1. *J Exp Med,* 1996, vol. 184, 1101-1109
- **DIEHL A M ; RAI R M.** Liver regeneration 3: Regulation of signal transduction during liver regeneration. *FASEB,* 1996, vol. 10 (2), 215-27

- **DRIZE N ; CHERTKOV J ; SAMOILINA N ; ZANDER A.** Effect of cytokine treatment (granulocyte colony-stimulating factor and stem cell factor) on hematopoiesis and the circulating pool of hematopoietic stem cells in mice. *Exp Hematol,* 1996, vol. 24, 816-822
- **DUHRSEN U ; VILLEVAL JL ; BOYD J ; KANNOU-RAKIS G ; MORSTYN G ; METCALF D.** Effects of recombinant human granulocyte colony-stimulating factor on hematopoietic progenitor cells in cancer patients. *Blood,* 1988, vol. 72, 2074-2081
- **GRISHAM ; THORGEIRSSON.** Stem Cells. Academic Press, 1997
- **KIM ; BROXMEYER.** In vitro behavior of hematopoietic progenitor cells under the influence of chemoattractants: stromal cell-derived factor-1, steel factor, and the bone marrow environment. *blood,* 1998, vol. 1, 100-110
- **KIM CH ; BROXMEYER HE.** SLC/exodus2/6Ckine/TCA4 induces chemotaxis of hematopoietic progenitor cells: differential activity of ligands of CCR7, CXCR3, or CXCR4 in chemotaxis vs. suppression of progenitor proliferation. *J Leuk Biol,* 1999, vol. 66, 455
- **KOLLET 0 ; SPIEGEL A ; PELED A ; PETIT I ; BYK T ; HERSHKOVIZ R ; GUETTA E ; BARKAI G ; NAGLER A ; LAPIDOT T.** Rapid and efficient homing of human CD34(+)CD38(-/low)CXCR4(+) stem and progenitor cells to the bone marrow and spleen of NOD/SCID and NOD/SCID/B2m(null) mice. *Blood,* 2001, vol. 97, 3283-91
- **LAGASSE et al.** Purified hematopoietic stem cells can differentiate into hepatocytes in vivo. *Nature medicine,* 2000, vol. 6, 1229-34
- **LAGASSE et al.** Toward regenerative medicine. *Immunity,* vol. 14, 425-36

- **LAPIDOT.** Mechanism of human stem cell migration and repopulation of NOD/SCID and B2mnull NOD/SCID mice. The role of SDF-1/CXCR4 interactions. *Ann. NY Acad. Sci.,* 2001, vol. 938, 83-95
- **LOTTI et al.** Transcriptional targeting of lentiviral vectors by long terminal repeat enhancer replacement. *Journal of Virology,* 2002, vol. 76 (8), 3996-4007
- **MAZO IB ; VON ANDRIAN UH.** Adhesion and homing of blood-borne cells in bone marrow microvessels. *Journal of leukocyte Biology,* 1999, vol. 66, 25-32
- **NOVELLI, M. et al.** Polyclonal origin of colonic adenomas in an XO/XY patient with FAP. *Science,* 1996, vol. 272, 1187-1190
- **NOVIKOFF PM ; YAM A ; OIKAWA I.** Blast-like cell compartment in carcinogen-induced proliferating bile ductule. *Am J Pathol,* May 1996, vol. 148 (5), 1473-92
- **PAPAYANNOPOULOU T.** Hematopoietic stem/progenitor cell mobilization. A continuing quest for etiologic mechanisms. *Ann N Y Acad Sci,* 1999, vol. 872, 187-197discussion 197-9
- **PELED A ; PETIT I ; KOLLET 0 ; MAGID M ; PONOMARYOV T ; BYK T ; NAGLER A ; BEN-HUR H ; MANY A ; SHULTZ L.** Dependence of human stem cell engraftment and repopulation of NOD/SCID mice on CXCR4. *Science,* 1999, vol. 283, 845-848
- **PELED A ; GRABOVSKY V ; HABLER L ; SANDBANK J ; ARENZANA-SEISDEDOS F ; PETIT I ; BEN-HUR H ; LAPIDOT T ; ALON R.** The chemokine SDF-1 stimulates integrin-mediated arrest of CD34(+) cells on vascular endothelium under shear flow. *The Journal of Clinical Investigation,* 1999, vol. 104, 1199-1211
- **PETERSEN et al.** Bone marrow as a potential source of hepatic oval cells. *SCIENCE,* 1999, vol. 284, 1168-70
- **PONOMARYOV T ; PELED A ; PETIT I et al.** Induction of the chemokine stromal-derived factor-1 following DNA damage improves human stem cell function. *J Clin Invest.,* 2000, vol. 106, 1331-1339
- **ROSU-MYLES M ; GALLACHER L ; MURDOCH B ; HESS DA ; KEENEY M ; KELVIN D ; DALE L ; FERGUSON SS ; WU D ; FELLOWS F.** The human hematopoietic stem cell compartment is heterogeneous for CXCR4 expression. *PNAS,* 2000, vol. 97, 14626-14631
- **SIENA S ; BREGNI M ; BRANDO B ; RAVAGNANI F ; BONADONNA G ; GIANNI AM.** Circulation of CD34+ hematopoietic stem cells in the peripheral blood of high-dose cyclophosphamide-treated patients: enhancement by intravenous recombinant human granulocyte-macrophage colony-stimulating factor. *Blood,* 1989, vol. 74, 1905-1914
- **STROM SC ; FISHER RA ; THOMPSON MT ; SANYAL AJ ; COLE PE ; HAM JM ; POSNER MP.** Hepatocyte transplantation as a bridge to orthotopic liver transplantation in terminal liver failure. *Transplantation,* 27 February 1997, vol. 63 (4), 559-69
- **SUZUKI et al.** Loss of SDF-1 receptor expression during positive selection in the thymus. *Intern Immunol.,* 1998, vol. 10 (8), 1049-1056
- **SWEENY, E.A. ; PRIESTLEY, G. ; NAKAMOTO, B. ; PAPAYANNOPOULOU, T.** Sulfated Polysaccharides Increase Plevels of SDF-1 in Monkeys and Mice: Involvement in Mobilization of Stem/Progenitor Cells. *Abstracts of the 42nd annual meeting of the American society of Haematology,* 01 December 2000
- **TANAKA et al.** Fetal microchimerism alone does not contribute to the induction of primary biliary cirrhosis. *Hepatology,* 1999, vol. 30, 833-838
- **VAN EYKEN.** Cytokeratins and the liver. *Liver,* 1993, vol. 13, 113-22
- **WEGLARZ TC ; DEGEN JL ; SANDGREN EP.** Hepatocyte transplantation into diseased mouse liver. 2000 Dec;Kinetics of parenchymal repopulation and identification of the proliferative capacity of tetraploid and octaploid hepatocytes. *Am J Pathol.,* vol. 157 (6), 1963-74
- **WOLFE et al.** Isolation and characterization of an alphoid centromeric repeat family from the human Y chromosome. *J Mol Biol.,* 1985, vol. 182, 477-485
- **ZHENG et al.** Genomic integration and gene expression by a modified adenoviral vector. *Nat Biotechnology,* 2000, vol. 18, 176-180